# EUROPEAN PATENT APPLICATION

(11) **EP 1 935 328 A2**
(43) Date of publication of application: **25.06.2008**
(21) Application number: 07253684.0
(22) Date of filing: 18.09.2007
(51) Int. Cl.: A61B 1/24

(54) **Illumination apparatus**

(30) Priority: 05.10.2006 GB 0619658
(71) Applicant: Optident Limited, Valley Drive Ilkley LS29 8PB (GB)
(72) Inventor: Fowler, James, Ilkley LS29 8PB (GB)
(74) Representative: Tomkinson, Alexandra

(57) **Abstract**

Illumination apparatus is provided including a first member having attachment means associated therewith for allowing attachment of the apparatus to an item of equipment, such as a camera requiring illumination. At least one housing is associated with said first member. The housing has an illumination source associated therewith so as to provide the illumination. The illumination source of said housing includes fibre optic means.

## Description

This invention relates to illumination apparatus.

Although the following description refers almost exclusively to illumination apparatus for use on a camera, and particularly to apparatus for use on a camera in a dental application, it will be appreciated by persons skilled in the art that the illumination apparatus of the present invention can be used on any suitable equipment for any suitable application, such as on a microscope and/or the like.

Illumination of teeth in dental applications is important, particularly in dental restoration applications where the colour of the material being used to repair the teeth has to be closely matched with the patient's teeth being repaired. Conventionally, such colour matching is performed by taking the patient to a daylight source, such as a window and matching a ceramic or other repair material with the patient's teeth. The technician or dentist undertaking this procedure will try to obtain a light source of a particular daylight standard, such as for example 5500 Kelvin (measure of colour temperature) which is generally held to be a standard which most closely corresponds to "noon" daylight or the level of daylight at the brightest point in a day. However, it will be appreciated that this method is susceptible to error and is typically dependent on factors such as the time of day at which the colour matching is undertaken, the amount of daylight available and/or the like. In addition, it is inconvenient to have to restrict colour matching to particular times of the day. If the colour matching is incorrect the patient may have dentures, fillings and/or the like which do not match the remainder of their teeth, which can be aesthetically displeasing and distressing to the patient.

In addition to the shade matching process, dentists provide dental laboratories with digital photographs which help to communicate the appearance of the restoration. The lighting level in the locality at the time at which the photograph is taken will significantly alter the colour of the teeth in the resulting image, thereby making colour reproduction inconsistent. In an attempt to overcome this problem, it is known to provide lighting apparatus which is detachably mounted to the front of the lens of a camera to provide a continuous and controllable light, such as for example of the type produced by the company DoctorsEyes™ to try to ensure reproducibility of light and consistency of results. More particularly, the lighting apparatus includes a central ring light which is fitted to the front of the lens with two lateral light panels mounted at acute angles on opposite sides of the ring light. The ring light and lateral light panels include a plurality of rows of white LEDs, the brightness of which can be controlled to produce a required level of lighting for photography. However, problems associated with this type of device is that the LEDs do not typically provide lighting over the correct spectrum necessary to achieve a colour rendering index sufficient to obtain colour reproducibility.

It is an aim of the present invention to provide improved illumination apparatus.

It is a further aim of the present invention to provide a camera including improved illumination apparatus.

It is a yet further aim of the present invention to provide a method of using improved illumination apparatus.

According to a first aspect of the present invention there is provided Illumination apparatus, said illumination apparatus including a first member having attachment means associated therewith for allowing attachment of the apparatus to an item of equipment requiring illumination, and at least one housing associated with said first member, said housing having an illumination source associated therewith so as to provide the illumination, and wherein said illumination source of said housing includes fibre optic means.

Preferably the at least one housing is provided laterally of said first member. For example, the at least one housing protrudes outwardly from a side surface of said first member.

Preferably the at least one housing is located at an acute angle to the first member and/or to at least a part of the item of equipment to which the apparatus is to be attached. Further preferably the at least one housing is located at an angle of approximately 45 degrees to the item of equipment and/or first member.

Preferably at least part of the at least one housing is movable relative to said first member. The at least part of the at least one housing can be movably mounted to the first member or can be movably mounted relative to a remaining part of said housing.

The at least one housing typically includes at least one aperture to allow the light emitted from the fibre optic means in use to exit the housing. The at least one aperture is preferably located at a front of the housing.

Preferably the at least one aperture is in the form of an elongate slot and further preferably the longitudinal axis of the elongate slot is arranged substantially horizontally of the housing. For example, substantially parallel to the longitudinal axis of the housing.

In one embodiment a portion of the housing in which the at least one aperture is located is movable relative to a remaining part of the housing.

Preferably said movable portion is rotatably or slidably movable relative to a remaining part or parts of the housing or first member.

The movable portion can preferably move substantially horizontally and/or vertically through any required angle. For example, the movable portion can be moved about an axis substantially perpendicular to the longitudinal axis of the apparatus in one embodiment. Two or more housings can be moved through the same and/or different angles as required.

The housing preferably includes at least one further opening for the location of at least one fibre optic cable therethrough. This opening is typically located at a back of the housing and/or opposite to the at least one aperture through which the light from the fibre optic is emitted from.

One or more lenses can be provided in or associated with the at least one housing to allow focusing of the light emitted therefrom. In one embodiment a cylindrical lens is provided.

In one embodiment two housings are provided on the apparatus, each housing located on opposite sides of the first member to provide two lateral housings. Further housings can be provided adjacent the first member if required.

Preferably indication means are associated with the at least one housing and/or first member to allow a user to determine the angle and/or position of the movable portion of the housing relative to a remaining portion of the housing and/or first member. The indication means allows a user to be able to reproduce the lighting conditions for a particular application, thereby providing accurate and reproducible results.

The indication means can be a visual and/or audio indication, such as a clicking sound on movement of the movable portion and/or visual markings provided on a part of the housing.

Preferably a light source is provided remotely of said illumination apparatus to which a distal end of the fibre optic cable is associated with. The light source is typically provided in its own housing or light box and can include any suitable light generating means, such as high intensity discharge lamp. One or more filters and/or lens can be associated with the remote light source housing if required to filter out harmful radiation, such as ultraviolet light and/or infra-red light, and/or focus the light in a suitable manner.

Cooling means can be associated with the remote light source housing and/or on the at least one housing of the apparatus to ensure the apparatus does not get too hot during use.

The light emitted from the fibre optic cable is typically at a colour temperature of approximately 5500 Kelvin and/or typically corresponding to "noon daylight".

In one embodiment the first member is substantially annular in form. The annular first member is typically fitted or adapted to be fitted to the front of a camera lens so that the at least one illumination housing is located laterally of the lens.

Illumination means can be associated with the first member as well as the one or more lateral housings if required. Thus, in one example, fibre optic lighting means can be provided on or associated with the first member.

The attachment means can include any suitable attachment including any or any combination of one or more screw threads, friction fit, one or more clips, ties, nuts and bolts, inter-engaging members, welding, magnetic means and/or the like.

In one embodiment the first member includes at least first and second member parts. The first part can be attached directly to the item of equipment and the second part can be attached to the first part. The one or more housings are typically associated with the second part.

The first and second parts can be detachably attached as required.

In a preferred embodiment, the first part is attached to the item of equipment, such as for example to the front of a camera lens, via a screw thread arrangement. The second part is attached to the first part via magnetic means.

Preferably the magnetic means includes one or more magnets and further preferably rare earth magnets, such as neodymium magnets.

According to a second aspect of the present invention there is provided a camera including illumination apparatus.

According to a further aspect of the present invention there is provided method of using illumination apparatus, said method including the steps of attaching a first member of said illumination apparatus to an item of equipment requiring illumination, at least one housing associated with said first member, providing an illumination source in said housing so as to provide the illumination, and wherein said illumination source of said housing includes fibre optic means.

According to a yet further aspect of the present invention there is provided illumination apparatus, said illumination apparatus including a first member having attachment means associated therewith for allowing attachment of the apparatus to an item of equipment requiring illumination, and at least one housing located laterally of said first member, said housing having an illumination source associated therewith so as to provide illumination, and wherein said illumination source of said lateral housing includes fibre optic means.

The illumination apparatus of the present invention has the advantage that it is easy to use and provides substantially continuous and consistent illumination at the required colour temperature suitable for colour rendering. By providing continuous reproducible lighting, a photographer can set up the camera using desired settings much more reliably than using a flash since they can see the subject being photographed clearly prior to the image being captured. The apparatus also minimises specular reflection. The illumination apparatus can be used for any medical or macro-photography application and/or the like.

An embodiment of the present invention will now be described with reference to the accompanying figures:

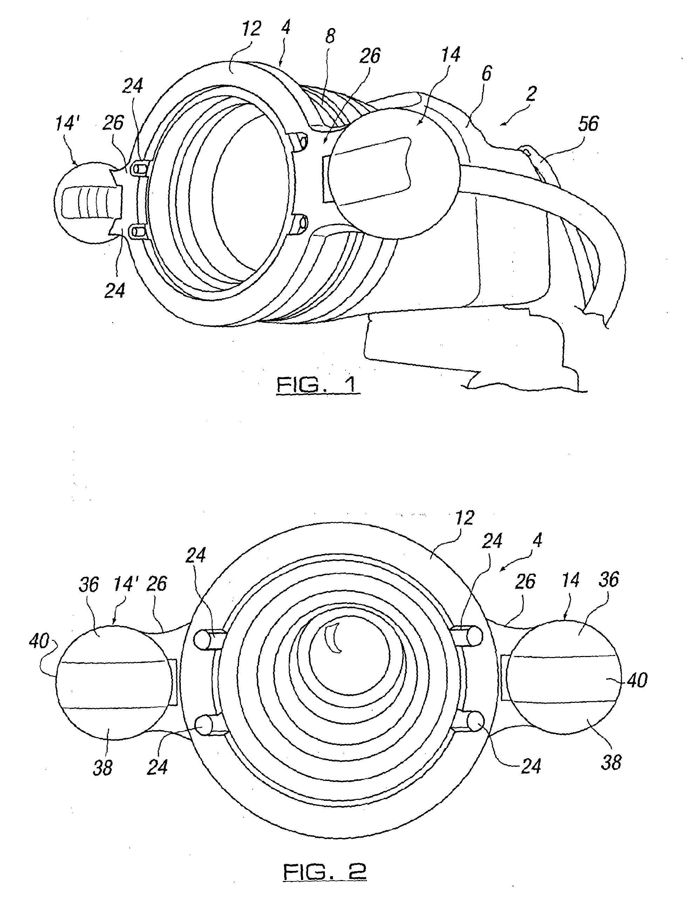

Figure 1 is a perspective view of the illumination apparatus fitted to a camera in use;
Figure 2 is a front view of the illumination apparatus in figure 1;

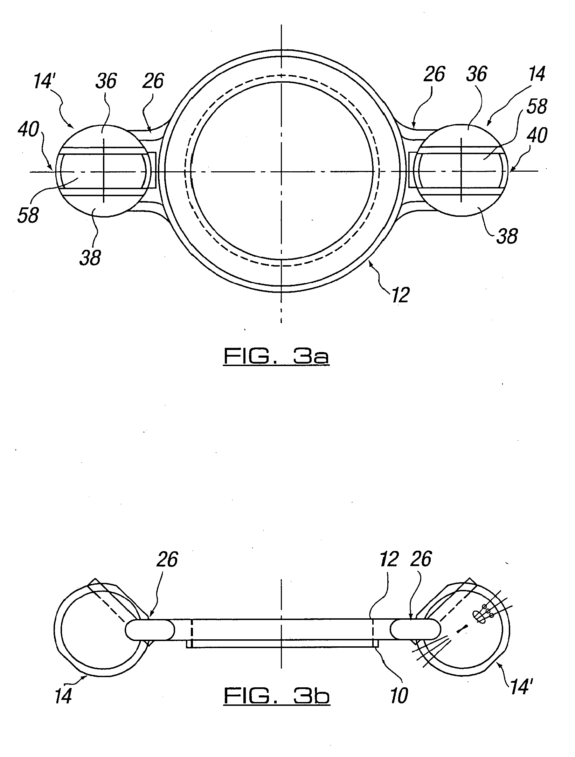

Figures 3a and 3b show a front view and plan view from above of the illumination apparatus respectively;
Figures 4a-4c illustrate a cross sectional view, front view and plan view from above of an optical housing of the illumination apparatus;
Figures 5a-5c illustrate a cross sectional view, front view and plan view from above respectively of the optical block forming part of the optical housing in figures 4a-4c;
Figures 6a-6c illustrate a plan view from below and side views respectively of the optical cap forming part of the optical housing in figures 4a-4c;
Figures 7a-7b illustrate a cross sectional view and front view respectively of a first lens adaptor ring;
Figures 8a-8b illustrate a side view and rear view of a second lens adaptor ring for fitting to the first lens adaptor ring shown in figures 7a-7b; and
Figures 9a-9b illustrate a front view and side view respectively of the mounting bracket for mounting the optical housing in figures 4a-4c to the further adaptor ring in figures 8a-8b.

Referring to the figures, there is illustrated a digital camera 2 having illumination apparatus 4 associated therewith to provide continuous and consistent lighting to allow accurate colour rendering for a dental application.

The digital camera 2 includes a camera body 6 and a lens arrangement 8 located on a front of the camera body. The illumination apparatus 4 is attached to a front of the lens arrangement 8 in use.

The illumination apparatus includes a first member comprising first and second member parts; a first lens adaptor ring part 10 and a second lens adaptor ring part 12 for detachable attachment to adaptor ring 10. Two lateral optical housings 14, 14' are provided on opposite sides of lens adaptor ring 12.

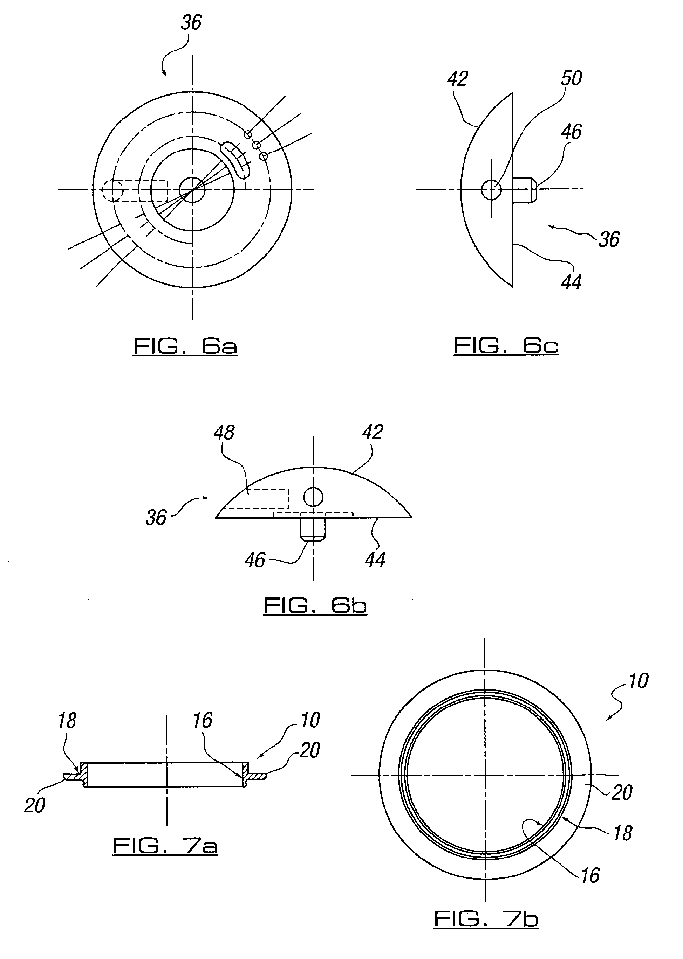

The first lens adaptor ring 10 has an inner surface 16, as shown in figures 7a-7b, having a screw thread provided thereon to allow adaptor ring 10 to be screwed to the front of the lens arrangement 8 in use, and an outer surface 18. A peripheral outwardly protruding flange 20 is provided on outer surface 18 to which the second lens adaptor ring 12 engages to when assembled.

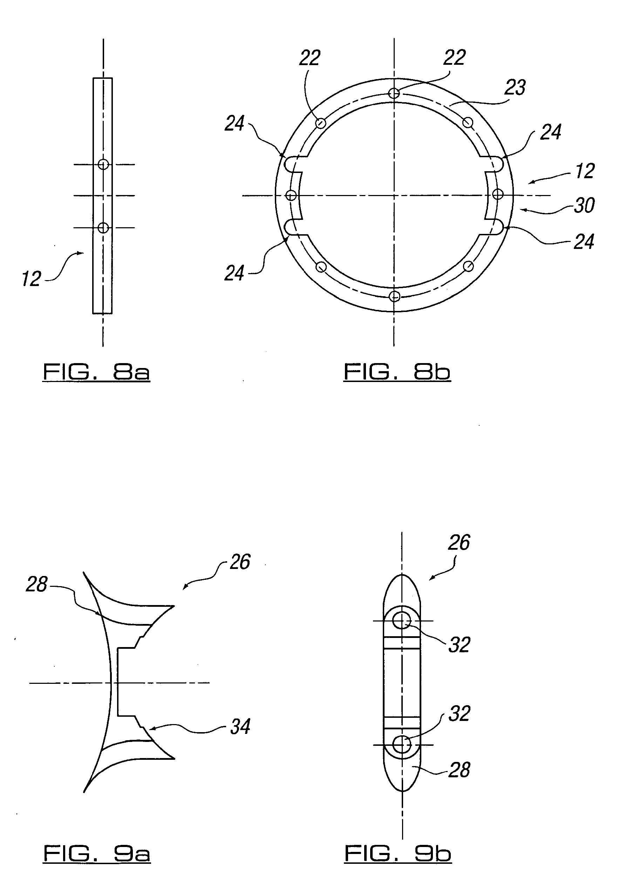

The second lens adaptor ring 12, as shown in figures 8a-8b has a plurality of rare earth magnets 22 provided at spaced apart intervals on a rear surface 23 of the ring. These magnets allow detachable attachment of the adaptor ring 12 to flange 18 of adaptor ring 10. The first lens adaptor ring 10 is preferably formed from stainless steel or some other ferrous or magnetic material having opposite polarity to magnets 22 to allow engagement between adaptor ring 12 and adaptor ring 10.

Two recesses 24 are defined on each side of ring 12 at opposite locations to allow screw attachment of optical housings 14, 14' thereto via mounting bracket 26. More particularly, mounting bracket 26, as shown in figures 9a-9b, has a first arc shaped end 28 for location against peripheral outer edge 30 of ring 12 and two screw apertures 32 provided at spaced apart intervals on end 28 to allow screw attachment to ring 12. An opposite end 34 of mounting bracket 26 is substantially concave in shape to allow location of part of complementary shaped spherical optical housing 14, 14' therein.

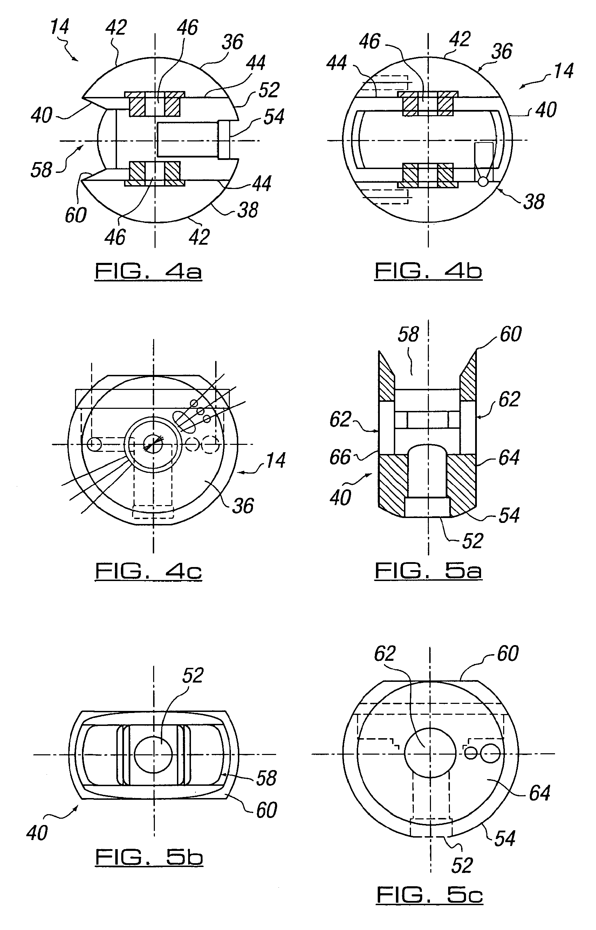

Each optical housing 14, 14' is substantially spherical in shape and includes top and bottom optical caps 36, 38, as shown in figures 4a-4c and 6a-6c, with an optical block 40, as shown in figures 4a-4c and 5a-5c, rotatably mounted therebetween.

Each optical cap 36, 38 is substantially hemispherical in shape with a curved outer surface 42 and a substantially planar inner surface 44. A rotation shaft 46 is provided substantially centrally of planar inner surface 44. Shaft 46 typically protrudes outwardly of surface 44. A channel 48 having an opening 50 on outer surface 42 allows attachment of the optical cap to end 34 of mounting bracket 26. The optical caps 36, 38 are typically attached to mounting bracket so as to be at an angle of approximately 45 degrees to mounting bracket 26 and/or adaptor ring 12. This 45 degree angle has been found to produce the best colour rendering index.

Optical block 40 has an opening 52 in rear surface 54 thereof to allow location of an end of a fibre optic cable 56 therethrough in use. An elongate slot 58 is defined in front surface 60 of optical block 40 and is orientated substantially horizontally when the optical housing 14 is fitted to adaptor ring 12. Slot 58 is provided substantially opposite to opening 52 so as to allow light emitted from the end of fibre optic cable 56 to pass through said slot.

Optical block 40 has apertures or recesses 62 provided on a top surface 64 and base surface 66 to allow rotation shaft 46 of the top and bottom caps 36, 38 to be rotatably mounted therein. When assembled, optical block can be rotated about a substantially vertical axis, typically between 0-180 degrees, to allow elongate slot 58 to be angled at any suitable angle relative to the lens arrangement 8. This allows the light beam emitted from optical housing 14 to be focused onto a suitable location as required.

The light source associated with fibre optic cable 56 is typically located remotely from camera 2 and is located in a conventional light box housing a POV™ high intensity discharge light. This light can be filtered for ultraviolet and infra red elements in the light box. Thus, a first end of fibre optic cable 56 is located in the remote light box, this cable 56 then splits into two separate cables to allow second ends to be attached to optical housings 14 and 14' respectively. Light passes from first end of cable 56, along the cable to be emitted at the second end in housings 14, 14'.

A lens arrangement can be associated with elongate slot 58 in optical block 40 to allow the light emitted from the second end of the fibre optic cable to be focused in a suitable manner.

In use of the illumination apparatus, the first adaptor ring 10 is screwed to the front of the camera lens arrangement, the second adaptor ring 12 is magnetically attached to the front of ring 10 and the optical block is angled to a suitable angle depending on how far the subject being illuminated is from the camera. Indication means associated with the optical housing allows a user to set the position of the housing and thus the light beam emitted from the housing at a required angle. As such, a user can use pre-defined camera settings, a pre-defined angle of light at a pre-defined colour temperature to provide accurate and reproducible results.

Control means can be associated with the remote light source and/or optical housing to allow control of the light emitted from the light source in use.

## Claims

1. Illumination apparatus, said illumination apparatus including a first member having attachment means associated therewith for allowing attachment of the apparatus to an item of equipment requiring illumination, and at least one housing associated with said first member, said housing having an illumination source associated therewith so as to provide the illumination, and wherein said illumination source of said housing includes fibre optic means.

2. Illumination apparatus according to claim 1 wherein the at least one housing is located laterally of said first member.

3. Illumination apparatus according to claim 1 wherein the at least one housing is located at an acute angle to the first member and/or to at least part of said item of equipment.

4. Illumination apparatus according to claim 3 wherein the acute angle is approximately 45 degrees.

5. Illumination apparatus according to claim 1 wherein the at least part of the at least one housing is movable relative to said first member.

6. Illumination apparatus according to claim 1 wherein the at least one housing includes at least one aperture located at a front of the housing for allowing light to be emitted from the fibre optic means.

7. Illumination apparatus according to claim 6 wherein the at least one aperture is in the form of a elongate slot and the longitudinal axis of the slot is located substantially parallel to the longitudinal axis of the apparatus.

8. Illumination apparatus according to claim 6 wherein a portion of the housing with said at least one aperture is movable relative to a remaining portion of said housing.

9. Illumination apparatus according to claim 1 wherein one or more lenses are provided in or associated with said at least one housing to allow focusing of light emitted therefrom.

10. Illumination apparatus according to claim 7 wherein a cylindrical lens is used.

11. Illumination apparatus according to claim 1 wherein two housings are associated with the first member, each housing located on an opposite side of the first member.

12. Illumination apparatus according to claim 1 wherein indication means are associated with the at least one housing and/or first member to allow a user to determine the angle and/or position of the movable portion of the housing relative to a remaining portion of the housing and/or first member.

13. Illumination apparatus according to claim 1 wherein a light source is provided remotely of said illumination apparatus to which a distal end of a fibre optic cable is associated with, said remote light source provided in a further housing or light box.

14. Illumination apparatus according to claim 1 wherein the light emitted from the fibre optic cable is at a colour temperature of approximately 5500 Kelvin or corresponding to "noon daylight".

15. Illumination apparatus according to claim 1 wherein the item of equipment is a camera lens.

16. Illumination apparatus according to claim 1 wherein the first member is substantially annular in form.

17. Illumination apparatus according to claim 1 wherein the attachment means include any or any combination of one or more screw threads, friction fit, one or more clips, ties, nuts and bolts, inter-engaging members, welding and/or magnetic means.

18. Illumination apparatus according to claim 1 wherein the first member includes at least first and second member parts, the first member part attached directly to the item of equipment and the second member part attached to the first member part.

19. Illumination apparatus according to claim 18 wherein the at least one housing is associated with the second member part.

20. Illumination apparatus according to claim 18 wherein the first member part is detachably attached to the second member part.

21. Illumination apparatus according to claim 18 wherein the first member part is detachably attached to the second member part via magnetic means.

22. A method of using illumination apparatus, said method including the steps of attaching a first member of said illumination apparatus to an item of equipment requiring illumination, at least one housing associated with said first member, providing an illumination source in said housing so as to provide the illumination, and wherein said illumination source of said housing includes fibre optic means.

23. An item of equipment in the form of a camera with illumination apparatus attached thereto according to claim 1.
